# EUROPEAN PATENT SPECIFICATION

(11) **EP 3 206 571 B1**
(45) Date of publication and mention of the grant of the patent: **25.03.2020**
(21) Application number: 15730848.7
(22) Date of filing: 29.04.2015
(51) Int. Cl.: A61B 5/021, A61B 5/022, A61B 5/00, A61B 5/024, A61B 5/0285

(54) **DEVICE AND METHOD FOR MEASURING BLOOD PRESSURE AND PULSE WAVE OF A CAROTID ARTERY**
VORRICHTUNG UND VERFAHREN ZUR MESSUNG DES BLUTDRUCK UND DER PULSWELLE EINER KOPFSCHLAGADER
DISPOSITIF ET PROCÉDÉ DE MESURE DE PRESSION SANGUINE ET D'ONDE DE POULS D'UNE ARTÈRE CAROTIDE

(30) Priority: 13.10.2014 LT 2014118
(43) Date of publication of application: 23.08.2017
(73) Proprietor: Klaipeda University, 92294 Klaipeda (LT)
(72) Inventor: STANKUS, Albinas, LT-00138 Palanga (LT); LUKOSIUS, Zydrunas, LT-96394 Klaipedos raj. (LT)
(74) Representative: Klimaitiene, Otilija
(86) International application number: PCT/IB2015/053113
(87) International publication number: WO 2016/059482

(56) References cited:
- EP-A1- 1 338 242
- EP-A2- 1 325 705
- CN-A- 101 496 718
- JP-A- 2000 060 810

## Description

### Field of the Invention

The invention is related to field of medical devices and method of use thereof in cardiology, neurology, and blood research, in particular the invention is related to device, system and method for measuring blood pressure in a region of the carotid artery.

### Technical background

Nowadays more and more significance is attributed to measurement of blood pressure pulse wave in carotid aorta. According to research this method has a lot of advantages compared to blood pressure measurement in the arm area by Korotkov method.

Late pulse wave amplitude has significant importance. It is partially formed by peripheral resistance magnitude and measured only directly in aorta. When measuring pulse wave of blood pressure ratio between amplitudes of early and late systolic waves is calculated, i.e. index of augmentation which has greater diagnostic value then the Korotkov method, especially when considering the hypertonic disease. Conventional devices and methods for measuring diastolic blood pressure are based on known laws for direct relation of diastolic pressure with pulse wave velocity.

Patent application EP1325705 discloses an apparatus for measuring carotid artery blood pressure and pulse waves. Patent application No. 13/618,227 discloses a sensor system having plethysmographic sensors secured on a carotid artery and a temporal artery for measuring pulse wave velocity there between. Similar system is disclosed in patent application No. CN20111425825 for measuring pulse wave velocity between neck and brain arteries. In most cases pulse wave velocity is measured between carotid and femoral arteries as disclosed in patent application WO2011/045806. All mentioned methods lack precision because they are based on measuring indirect volumetric changes in capillaries and possibly arteries, i.e. it is impossible to measure diastolic blood pressure by these methods.

Japanese patent application No. JP2004-294238 discloses a device and a method for measuring pulse wave in an ear area (basin of carotid artery) by photoplethysmography. Using the invention, growth of difference of amplitudes of early and late waves is established as a circulation index. This method is not accurate because it simulates a known augmentation index, which is calculated from data of blood pressure measurements in aorta.

US patents US 7,468,037 B2 and US 6,659,958 B2 discloses devices for measuring augmentation index. Operation of both devices is based on increasing pressure by inflating a cuff fixed on a person's arm and at the same time registering the pulse wave. The shortcoming is big measuring distance from aorta at the time of measuring. This means that when a pulse wave propagates in the direction of periphery it changes its form and accordingly distorts the real augmentation index.

The closes technical solution is disclosed in a patent application No. US 11/738, 447. The disclosed measuring device is used for measuring a pulse wave of a carotid aorta. The measurement is performed after increasing pressure by air input in an air chamber positioned on a person's neck and performing measurement of the change of the signal of the pressure sensor therein. Main disadvantage of the device originates from indirectly measuring pressure level of carotid artery in the air chamber. Additionally pressurized air inhibits the pressure part and distorts the wave form with its capacitance parameters. This device is only used for performing indirect measurement of carotid blood pressure.

In addition to the mentioned disadvantages, such carotid pulse wave measurement devices also have issues with securing said devices to a user. Pressing the sensor with a hand causes vibrations of the sensor which hinders stability of the pressure. A patent application No. CN 201010529061 discloses a device for fixing and regulating position of carotid artery pulse wave sensor. It has a C-shape holder with a slider for placing on a neck and is adjustable relatively to the neck by means of a telescopic muff. The holder is adjusted with a resilient handle. Shortcoming of this device consists in absence of direct measurement of pressing force of the sensor to the carotid artery wall and further pressing is not controlled. Additionally C-shaped collar may press the sensor to the carotid artery insufficiently at the time of measuring pulses increasing possibility of receiving a diminished measured signal.

Having in mind the importance of diagnostics it is evident that pulse wave form of a carotid artery is very similar to the pulse wave present in aortic arch which is measured only by invasive methods. The goal of the invention is to measure systolic and diastolic blood pressure and propagating pulse wave in a carotid artery by means of computer controlled mechanical device for calculating augmentation index.

### Short description of the invention

Object of the invention is a device and a system for measuring blood pressure and it's pulse wave in carotid artery for calculating augmentation index and inner lumen of carotid artery and the calculation method. The scope of the invention is defined in the appended claims.

### Short description of the drawings

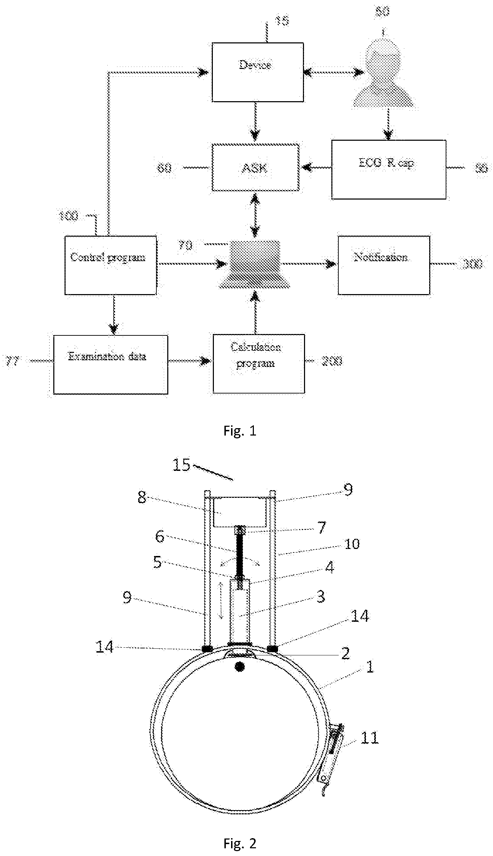

- Fig. 1 -: shows a block diagram of the system for measurement of carotid artery blood pressure and pulse wave and calculation of systolic and diastolic blood pressure, augmentation index and inner lumen of carotid artery;
- Fig. 2 -: shows the measurement device (15) for measuring blood pressure and its pulse wave of a carotid artery;

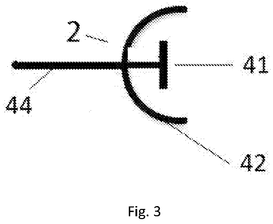

- Fig. 3 -: shows construction of a force sensor (2);

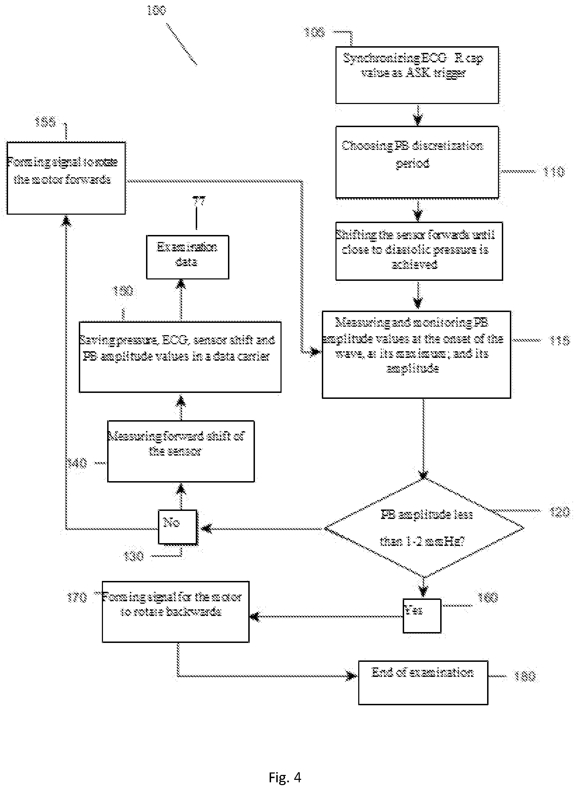

- Fig. 4 -: shows block diagram of the control program (100) of the device for measuring carotid artery blood pressure and its pulse wave;

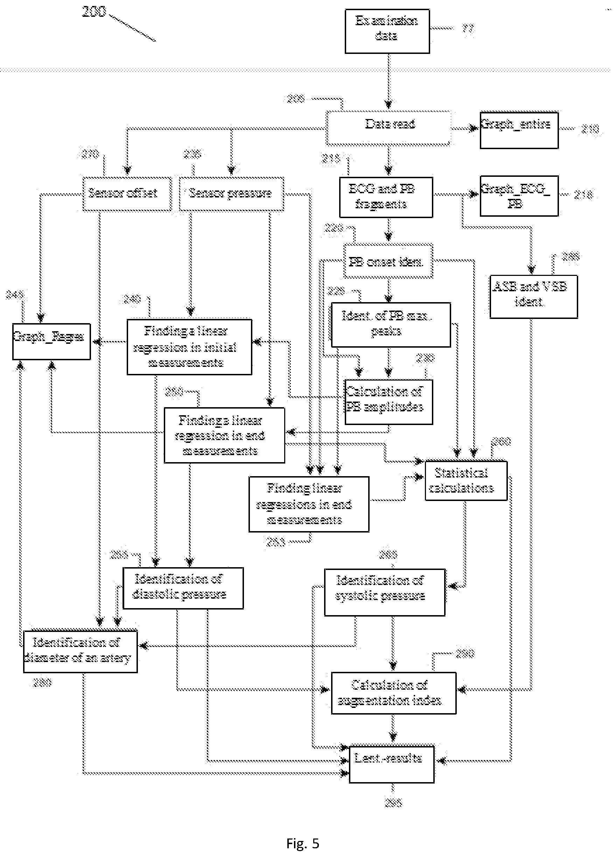

- Fig. 5 -: shows block diagram of the calculation program (200) for calculation of systolic and diastolic blood pressure, augmentation index and inner diameter of a carotid artery.

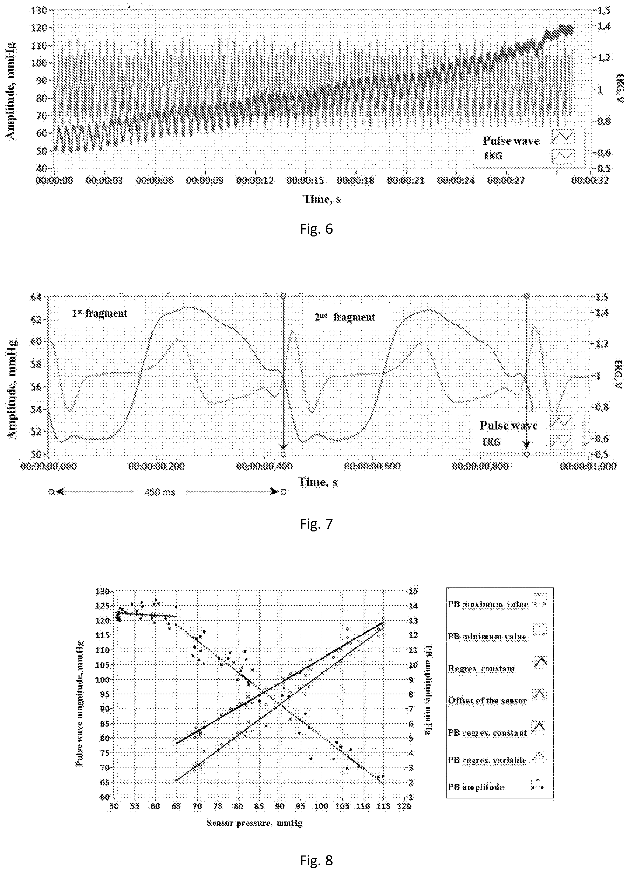

- Fig. 6 -: shows an exemplary electrocardiogram and dynamics of pulse wave of a carotid artery from the beginning of the examination to its end;
- Fig. 7 -: shows a fragment of analysis of electrocardiogram and pulse waves of carotid artery;
- Fig. 8 -: shows a graphical representation of analysis results of carotid artery pressure measurements;

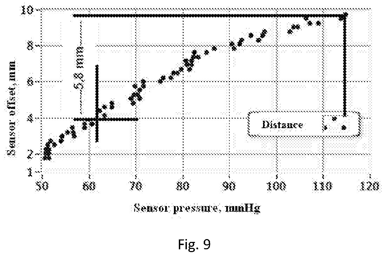

- Fig. 9 -: shows a graphical representation of analysis of measurement results of inner diameter of carotid artery pressure measurements.

### Description of the invention

Measuring system for measuring carotid artery blood pressure and its pulse wave comprises a device (15) comprising at least one securing member (1) of the device (15), i.e. ring shaped securing device with a slit for mounting the device (15) to a body part of a user (50). The arterial blood pressure is measured, so that the device (15) does not move with respect to the user's body part.The securing member (1) may be made of any rigid material for example, rigid plastic, so that when it is put on a body part, such as a neck, it temporarily deforms, where the securing member (1) is locked in on a patient's body using joining means (11); an actuator (10) comprising a computer program controlled motor (8), for example a stepper motor or a servo motor, rotatable elongated element (6), having a thread on at least part of its outer surface, a holder (7), for fixing the rotatable element (6) to the motor (8) shaft so that the shaft turns the rotatable elongated element (6); guiding spacer (5) for delimiting sideways motion_of the rotatable elongated element (6); a guiding member (4), rigidly fixed to the securing member (1), to which the spacer (5) is attached, and which at least partially comply with the form of the at least one reversably movable means (3) in order to be able to smoothly move reversably in a path restricted by the guiding member (4), where the guiding member (4) is rigidly attached to the at least one securing member (1); at least one reversably movable means (3), moving in a path defined by the guiding member (4), and where the rotatable element (6) rotating in a spacer (5) drives the reversably movable means (3) so that would move forward or backward in the direction of rotation of the motor. The actuator (10) also includes retainers (9) of the programmatically controllable motor (8) for rigidly mounting the motor (8) to a mounting point (14) of the at least one securing member (1).

The device also comprises a sensor (2), which includes at least a sensing element (41), which responds to the changes in pressure, convex-shaped element (42), at least partially enclosing the sensing element (41) which is rigidly fixed thereto, and a fixing part (44) for attaching the sensor to the reversably movable means (3).

When the measurements are performed in the neck carotid artery, the blood pressure and its pulse wave measuring system device (15) is affixed to the neck so that the sensor (2), such as the force sensor, would be at a minimum distance from the carotid artery, where the pressure sensing element (41), at least partially enclosed by the convex-shaped element (42) of the sensor (2), is connected with the reversably movable means (3) through a mounting part (44) of the sensor (2), so that when the sensing element (41) presses on the artery (13), the convex-shaped element (42) presses the artery so that it could not escape from the pressure point, where the distance between the edges of the convex-shaped element (42) is about 12 mm.

Carotid artery blood pressure and its pulse wave measuring system also includes a processor means (70) for the device (15), capable of carrying out at least the motor (8) control program (100), accept, process, store and control signals for the output device (15), and capable of executing calculation program (200) for calculating systolic and diastolic blood pressure, augmentation index and carotid artery inner diameter, and further comprising a computer readable data carrier (77).

The system also includes an analog to digital signal converter (ADC) (60) with the sampling frequency no less than 1000Hz. The ADC converter (60) is used to record mechanical pulsing forces of the pressed carotid artery wall and pulsation by using the sensor (2) and forward the recorded data to the computer readable data carrier (77) of the processor means (70), where additionaly an electrokardiogram (EKG) is used for control of the examination, where the cap R (55) is used as a trigger to start the process of discretization of EKG and pulse wave (PB).

The beginning of the examination is set from the size of the initial contact pressure of the pulse wave device (15), which initial contact pressure is found by measuring the blood pressure in an arm, using standard blood pressure measuring instruments in order to set the pressure magnitude less than the current size of the diastolics blood pressure in the arm. After the device (15) for measuring the carotid artery blood pressure and its pulse wave is locked on a neck using joining means (11) of the securing member (1), and the rotatable element (6) is rotated by a hand until the sensor (2) reaches the area for measuring the carotid artery pulsation, the control program (100) of the motor (8) of the device's (15) actuator (10) is initiated in the processing means (70). Using the motor (8) control program (100), the motor (8), which is controlled in such a way that the rotational velocity of the motor is minimum (e.g. in the case of a stepping motor-7.5 degrees per 1 step) is pressing on the sensor (2) via the turnable element (6) and the reversibly movable means (3) so that the beginning of each carotid artery pulse wave is registered together with pressure values of the sensing element (41) at said points. Pressure contact values are averaged at the beginning of each pulse wave and are stored in a computer readable data carrier (77). Using the control program (100) and depending on the heart rate frequency, the followeign are chosen: the observation period (110) of the processes of discretisation (EKG, and PB), which must be greater than the systole and equal to 350-500 ms, rotation steps and velocity of the motor, amplitude values of PB at its beginning and maximum part of the wave, and the difference between them, which is described as a PB amplitude, wherein said monitoring is performed in a display (115) which is connected to the processing means (115).

Computer readable data carrier (77) is used for storing the data of the averaged beginnings of the the pulse waves according to found pressure values by means of a special computer program (200) run on a processing means (70) which is used to calculate at least the amount of the offset of the sensor (2), pressing pressure, exact amplitude of the pulse waves and their forms, which are used to calculate the systolic and diastolic blood pressure, augmentation index and carotid artery inner diameter size. Obtained results are presented in the form of the notification (300). Said notification form (300) comprises at least the full results of the examination, e.g. in graphical form, an electrocardiogram (EKG) and PB snippet, a regresive dependencies graph and sizes, and a table of the measurements results, parameters of the operation of the device (15), such as motor (8) velocity of revolutions and the magnitude of the displacement of the reversably movable means (3).

Displacement of the reversably movable means (3) is controlled by controlling the motor (8) by a computer program so that the sensor (2) would reach initial pressure level, from the smaller initial disatolic pressure, of the carotid artery. Aferwards pulse waves are registered. The pressure by the sensor (2) is continouelsly increased by 3-5 mmHg. When this value is reached, the motor is stopped and at least three pulse waves of carotid artery are registered. From the at least three pulse waves the clamping pressure value with respect to values of PB amplitudes are calculated. Further on it is checked (120) whether the maximum amplitude of a pulse wave reached 1-2 mmHg pressure level. If this level is not reached (130), control program (100) sends a signal to the motor (8) to rotate forwards (155), at the same time measuring forward offset of the sensor (2) and pressure level over 3-5 mmHg until the end of the experiment. EKG, sensor (2) offset value and frequencies of PB amplitudes are stored (150) in computer readable data carrier (77). After this step, the operation is repeated until the value of the amplitude of PB become less than 1-2 mmHg (160), and after the value is reached (160), the control program (100) sends a signal to the motor to rotate backwards (170), and the examination is concluded.

After completion of the examination, the program for calculation (200) reads (205) the entire examination series from the computer readable data carrier (77) and submits them in the graph form (210). A graphical representation (218) is obtianed from the selected initial EKG and PB fragments (215). Based on said representation (218) PB time parameters are selected from the EKG and pulse wave structure, PB beginings (220) and the maximum peaks for acurate identification (225) from pulse wave structure. Based on them, differences of the amplitudes (PB amplitudes) (230) among all the found pulse waves beginings and the maximum PB values are calculated.

The next step comprises adaptive calculation of a linear regression dependency (240), between the sensore pressure values (235) red from the computer readable (77) and found values of the pulse waves amplitudes in the early stages of the examination by means of the the computing program (200) executed by the processor means (70), comprising increse up to the limit (RIBA), until the slope of the linear curve of the regresion dependency of the linear regression slope (slope) become negative. The number of measurement calculation is carried out in order to find the residual dispersion (S1) of measurements, which indicates accuracy of the measurement of the bottom (diastolic) pressure. From the pressure magnitude of said fixed sensor (2) regression coefficients of the linear dependence of the residual dispersion (S2) between the remaining pressure values and minimum values of the pulse waves are calculated. The same calculations (253) are performed between the values of the maximum amounts (225) of PB and the sensor (235) pressure values (235), where the linear regression dependence coefficient and the residual dispersion (S3) are derived . Both the regression dependecies are linear, so inevitably crosses at the vanishing point of the amplitude of the PB. The results of the two dependencies are represented graphically (245). These statistical values of the residual dispersio (S2 and S3) and obtained regresion coefficients allows identification of the limits of the sistolic pressure measurement error size (265). The size of the criteria is found (260) from previously found regresion coefficients and residual dispersions (S2, S3) (Brandt S. date Analysis in Statistical and computational methods for scientists and engineers. Springer Science & Business Media, 1999, 652s). When the difference between the maximum and minimum values of PB becomes smaller than this criteria, the pressure of the sensor becomes equivalent to the sistolic pressure, because it is believed that there was a reliable artery clamping. Pulse pressure (PP) is calculated from the sistolic and diastolic pressures.

Identification (285) of amplitude values of early (ASB) and late systolic waves (VSB) is performed from measured PB, up until fragments (215) of diastolic pressure moment (RIBA). Obtained pulse wave amplitudes show external tension of an artery, which is created the internal blood pressure rates. After using the obtained systolic and diastolic blood pressure values, ASB and VSB values are recalculated on the principle of proportionality to the sleep artery blood pressure units. Averages xA and xV and standard deviations sA and sV are calculated from obtained ASB and VSB pulse pressure values accordingly, where maximum value from the averages is determined and systolic pressure value (265) is assigned. If the maximum value belongs to the xA, in accordace with proportionality principle xA values, its standard deviations sA, xV, systolic blood pressure magnitude and its standard deviation sV are recalculated. Subsequntly Students criteria of reliability with probability less than 0,05 is used for determining statistical difference between xA and xV values (PsP) which is divided by PP and multiplied by 100. In this way, the result is the augmentation index Alx (290). If the maximum value belongs to xV, all values are recalculated in a similar fashion, and the resulting augmentation index Alx is with opposite sign than the found one, if xA was higher. If the statistical difference between xA and xV is unreliable, Alx is assumed to be zero. All the results of the calculation are shown in the table (295). Once the systolic and diastolic blood pressures are identified during the calculation, the distance difference due to the forward movement of the sensor, which indicates the size of the inner diameter of the artery in millimeters (280), is recorded.

## Claims

1. Carotid artery blood pressure and pulse wave measuring system comprising an arterial pressure sensor (2), a computer program controlled motor (8), and attachment means (1) for attaching the measuring device (15) to a user's neck, data processing means and an A/D converter (60) wherein the attachment means (1) being a rigid ring shaped with a slit for mounting the device (15) to a a user's neck; an actuator (10) comprising
- computer program controlled motor (8) controlled by a computer program (100) being stored in a computer readable data carrier (77) and run by the data processing means (70) being rigidly attached by retainers (9) to the attachment means (1) for attaching the measuring device to a user's neck,
- a rotatable elongated element (6), having a thread on at least part of its outer surface, at its one end attached to the shaft of the motor (8) by a holder (7) for fixing the elongated element (6) to the shaft of the motor (8) so that the elongated element (6) would be rigidly and axially attached to the shaft of the motor (8), and at its other end attached to a reversibly movable means (3) for reciprocating motion on a path between the motor (8) and the artery, wherein the elongated element (6) is configured to rotate in a spacer (5) for delimiting its sideways motion and wherein a guiding member (4) being rigidly attached to the attachment means (1) at least partially comply with the form of the at least one reversibly movable means (3); and
wherein the sensor (2) is rigidly attached to the free end of said reversibly movable means (3) for reciprocating motion on a path between the motor (8) and the artery and having a convex-shaped element (42) for preventing the artery from escaping from the pressure point; and
wherein said analog to digital signal converter (60) has a sampling frequency not less than 1000Hz for registration and transfer of the mechanical pulsation force.

2. The system according to claim 1 **characterised in that**, the computer program controllable motor (8) is a stepper motor.

3. The system according to claim 1 **characterised in that**, the computer program controllable motor (8) is a servo motor.

4. Method for measuring a carotid artery blood pressure and its pulse wave and calculating an augmentation index and carotid artery inner diameter using the system according to any one of the previous claims comprising the steps:
setting a contact pressure value of the pulse wave device (15) in order to set a contact pressure which is less than current diastolic blood pressure in an arm;
bringing a sensing element (41) of the sensor (2) to a carotid artery by turning the elongated element (6) of the device's (15) actuator (10) so that the sensing element (41) of the sensor (2) is positioned at minimum distance from the artery;
finding onset points of each pulse wave (PB) of the carotid artery and recording contact pressure values of the sensing element (41) at said onset points by using computer program controllable motor (8);
using a control program (100) choosing the monitoring time (110) of the sampling processes (EKG and PB), revolution velocity of the motor, value of PB amplitude at the onset of PB and at its maximum and difference between them, which is regarded as PB amplitude;
reaching smaller then diastolic pressure level at the contact with the sensor (2) which is primary pressure level reached from the initial pressure level and further increasing the pressure level to the value in the range of 3 to 5 mmHg;
stopping the motor (8) and recording at least three pulse waves of the carotid artery when said value in the interval from 3 to 5 mmHg is reached;
checking (120) whether maximum amplitude of the pulse wave reached 1-2 mmHg of the contact pressure level and sending a signal to the motor (8) by the control program (100) to rotate forward (155) if the contact pressure level is not reached (130) and measuring forward offset of the sensor (2) and contact pressure level through the range from 3 to 5 mmHg until end of examination;
repeating the operation until value of PB amplitude becomes less than 1-2 mmHg (160); sending a signal from the control program (100) to the motor (8) to rotate backwards (170) when said value (160) is reached;
sampling signals from the sensor (2) in analog to digital signal converter (60), where a cap (55) R or a cardiogram are used as a trigger for initiating sampling process of EKG and pulse wave (PB);
using a computer readable data carrier (77) for storing the data of the averaged beginnings of the pulse waves according to found pressure values by means of a special computer program (200) run on a processing means (70) which is used to calculate at least the amount of the offset of the sensor (2), pressing pressure, exact amplitude of the pulse waves and their forms, which are used to calculate the systolic and diastolic blood pressure, augmentation index and carotid artery inner diameter size and presenting the results in a form of a notification (300);
calculating the augmentation index and inner diameter of a carotid artery by means of calculation program (200), being executed in a processor means, by performing steps:
using the calculation program (200) to read (205) the entire examination series from the data carrier (77) and provide data in the form of a graph (210);
obtaining graphical representation (218) of the chosen initial EKG and PB fragments (215), using it to select its time parameters from EKG and pulse wave structure, PB onsets (220) and PB maximum peaks, where said time parameters are used to calculate PB amplitudes, which are differences (230) between all onsets and maximum PB values of pulse waves;
performing adaptive calculation of dependence of a linear regression (240), between the sensor values (235) from data carriers (77) and sizes of found amplitudes of pulse waves in early stages of examination, comprising calculating the measurement values, increasing up to the limit (RIBA), until the slope of regressive dependency curve becomes negative, performing said calculation to find ta remnant dispersion (S1) of the calculation, thereby defining the calculation accuracy of the diastolic pressure;
calculating (250) linear regression dependency coefficients and residual variance (S2) between the remaining contact pressure values and pulse wave minimum values from the sensor's (2) contact pressure magnitude (235);
performing calculations (253) between the values of the remaining maximum PB values (225) and the contact pressure values of the sensor (2), thereby obtaining linear regression dependency coefficients and a residual dispersion (S3);
graphically representing (245) dependencies results;
identifying systolic pressure value (265) according to statistical residual dispersion values S2 and S3 and obtained regressive measurement coefficients in measurement error margins;
finding (260) size of criteria from found regression coefficients and residual dispersions (S2, S3), where the differences between PB maximum values become less than said found value (260) of the criteria and the contact pressure magnitude is equal to systolic pressure;
calculating pulse pressure (PP) from systolic and diastolic pressure;
performing identification (285) of values of amplitudes of early systolic waves (ASB) and late systolic waves (VSB) from measured PB, up until fragments (215) of diastolic pressure moment (RIBA);
proportionally recalculating ASB and VSB values to the present carotid artery blood pressure units using found systolic and diastolic blood pressure values;
calculating averages xA and xV and standard deviations sA and sV from ASB and VSB pulse pressure values accordingly, from said averages finding maximum value, to which systolic pressure value (265) is accorded, where if the maximum value belongs to xA, value of xA, its standard deviations sA and xV, systolic blood pressure value and its standard deviation sV are recalculated proportionally;
looking for statistical difference between xA and xV values (PsP) using Student's reliability criteria with probability less than 0,05, where said difference is divided by PP and multiplied by 100, thereby obtaining augmentation index Alx (290);
representing all calculation results in a table (295);
recording a distance difference with respect to the forward offset of the sensor during the calculation and at the diastolic and systolic blood pressure values are identified.

## Patentansprüche

1. Karotisblutdruck- und Pulswellenmesssystem, umfassend einen Arteriendrucksensor (2), einen computerprogrammgesteuerten Motor (8) und Befestigungsmittel (1) zum Befestigen der Messvorrichtung (15) an einem Hals eines Benutzers, Datenverarbeitungsmittel und einen A/D-Wandler (60), wobei das Befestigungsmittel (1) ein starrer Ring ist, der mit einem Schlitz zum Montieren der Vorrichtung (15) an einem Hals eines Benutzer geformt ist;
einen Aktor (10), umfassend
- computerprogrammgesteuerten Motor (8), der von einem Computerprogramm (100) gesteuert wird, das in einem computerlesbaren Datenträger (77) gespeichert ist und von dem Datenverarbeitungsmittel (70) abgespielt wird, der durch Rückhaltemittel (9) starr an dem Befestigungsmittel (1) zum Befestigen der Messvorrichtung an einen Hals eines Benutzers befestigt ist,
- ein drehbares längliches Element (6), das ein Gewinde an mindestens Teil seiner Außenfläche aufweist, das an seinem einen Ende durch eine Halterung (7) an der Welle des Motors (8) befestigt ist, um das längliche Element (6) an der Welle des Motors (8) zu fixieren, sodass das längliche Element (6) starr und axial an der Welle des Motors (8) befestigt wäre, und an seinem anderen Ende an einem umgekehrt beweglichen Mittel (3) für hin- und herbewegende Bewegung auf einem Pfad zwischen dem Motor (8) und der Arterie befestigt ist, wobei das längliche Element (6) konfiguriert ist, in einem Abstandhalter (5) zu rotieren, um seine seitwärtige Bewegung zu begrenzen und wobei ein Führungselement (4), das starr an dem Befestigungsmittel (1) befestigt ist, mindestens teilweise der Form des mindestens einen umgekehrt beweglichen Mittels (3) entspricht; und
wobei der Sensor (2) starr an dem freien Ende des umgekehrt beweglichen Mittels (3) zum Hin- und Herbewegen auf einem Pfad zwischen dem Motor (8) und der Arterie befestigt ist und ein konvexförmiges Element (42) aufweist, das die Arterie daran hindert, dem Druckpunkt zu entkommen; und
wobei der Analog-Digital-Signalwandler (60) eine Abtastfrequenz von nicht weniger als 1000 Hz für Registrierung und Transfer der mechanischen Pulsationskraft aufweist.

2. System nach Anspruch 1, **dadurch gekennzeichnet, dass** der computerprogrammsteuerbare Motor (8) ein Schrittmotor ist.

3. System nach Anspruch 1, **dadurch gekennzeichnet, dass** der computerprogrammsteuerbare Motor (8) ein Servomotor ist.

4. Verfahren zum Messen eines Karotisblutdrucks und seiner Pulswelle und Berechnen eines Augmentationsindexes und Karotisinnendurchmessers unter Verwendung des Systems nach einem der vorstehenden Ansprüche, umfassend die Schritte:
Einstellen eines Kontaktdruckwerts der Pulswellenvorrichtung (15), um einen Kontaktdruck einzustellen, der weniger als aktueller diastolischer Blutdruck in einem Arm ist;
Bringen eines Erfassungselements (41) des Sensors (2) zu einer Karotis durch Drehen des länglichen Elements (6) des Aktors (10) der Vorrichtung (15), sodass das Erfassungselement (41) des Sensors (2) bei minimalem Abstand von der Arterie positioniert ist;
Ermitteln von Anfangspunkten jeder Pulswelle (PB) der Karotis und Aufzeichnungen von Kontaktdruckwerten des Erfassungselements (41) bei den Anfangspunkten unter Verwendung von computerprogrammsteuerbarem Motor (8);
Verwenden eines Steuerprogramms (100), um die Überwachungszeit (110) der Abtastprozesse (EKG und PB), Drehzahl des Motors, Wert von PB-Amplitude bei Anfang von PB und bei deren Maximum und Unterschied zwischen ihnen, der als PB-Amplitude betrachtet ist, zu wählen;
Erreichen von kleinerem als diastolischem Druckpegel bei dem Kontakt mit dem Sensor (2), der primärer Druckpegel ist, der vom Anfangsdruckpegel erreicht wird, und weiter Erhöhen des Druckpegels auf den Wert in der Spanne von 3 bis 5 mmHg;
Stoppen des Motors (8) und Aufzeichnen von mindestens drei Pulswellen der Karotis, wenn der Wert im Intervall von 3 bis 5 mmHg erreicht ist;
Prüfen (120), ob maximale Amplitude der Pulswelle 1-2 mmHg des Kontaktdruckpegels erreicht hat, und Senden eines Signals an den Motor (8) durch das Steuerprogramm (100), vorwärts zu drehen (155), falls der Kontaktdruckpegel nicht erreicht ist (130), und Messen von Vorwärtsversatz des Sensors (2) und von Kontaktdruckpegel über die Spanne von 3 bis 5 mmHg, bis zum Untersuchungsende;
Wiederholen des Betriebs, bis ein Wert von PB-Amplitude weniger als 1-2 mmHg wird (160);
Senden eines Signals vom Steuerprogramm (100) an den Motor (8), um rückwärts zu drehen (170), wenn der Wert (160) erreicht ist;
Abtasten von Signalen von dem Sensor (2) in Analog-Digital-Signalwandler (60), wo eine Kappe (55) R oder ein Kardiogramm als ein Trigger verwendet sind, um Abtastprozess von EKG und Pulswelle (PB) anzufangen;
Verwenden eines computerlesbaren Datenträgers (77) zum Speichern der Daten der gemittelten Ursprünge der Pulswellen gemäß ermittelter Druckwerte mittels eines Sondercomputerprogramms (200), das auf einem Verarbeitungsmittel (70) läuft, das verwendet ist, um mindestens die Menge des Versatzes des Sensors (2), Anpressdruck, exakte Amplitude der Pulswellen und deren Formen, die verwendet sind, um den systolischen und diastolischen Blutdruck zu berechnen, Augmentationsindex und Karotisinnendurchmessergröße zu berechnen und die Ergebnisse in einer Form einer Benachrichtigung (300) darzustellen;
Berechnen des Augmentationsindexes und Innendurchmessers einer Karotis mittels Berechnungsprogramm (200), das in einem Prozessormittel durchgeführt wird, durch Durchführen von Schritten:
Verwenden des Berechnungsprogramms (200), die gesamte Untersuchungsreihe vom Datenträger (77) zu lesen (205) und Daten in der Form eines Graphen (210) bereitzustellen;
Erhalten von grafischer Darstellung (218) der gewählten Anfangs-EKG- und PB-Fragmente (215), Verwenden derselben, um ihre Zeitparameter von EKG- und Pulswellenstruktur, PB-Anfängen (220) und PB-Maximalspitzen auszuwählen, wo die Zeitparameter verwendet sind, um PB-Amplituden zu berechnen, die Unterschiede (230) zwischen allen Anfängen und maximalen PB-Werten von Pulswellen sind;
Durchführen adaptiver Abhängigkeitsberechnung einer linearen Regression (240) zwischen den Sensorwerten (235) von Datenträgern (77) und Größen ermittelter Amplituden von Pulswellen in frühen Untersuchungsphasen, umfassend Berechnen der Messwerte, Erhöhen bis zur Grenze (RIBA), bis die Steigung regressiver Abhängigkeitskurve negativ wird, Durchführen der Berechnung, um eine restliche Dispersion (S1) der Berechnung zu ermitteln, dadurch Definieren der Berechnungsgenauigkeit des diastolischen Drucks;
Berechnen (250) linearer Regressionsabhängigkeitskoeffizienten und von Restvarianz (S2) zwischen den verbleibenden Kontaktdruckwerten und Pulswellenminimalwerten von der Kontaktdruckmagnitude (235) des Sensors (2);
Durchführen von Berechnungen (253) zwischen den Werten der verbleibenden maximalen PB-Werte (225) und den Kontaktdruckwerten des Sensors (2), dadurch Erhalten linearer Regressionsabhängigkeitskoeffizienten und einer restlichen Dispersion (S3);
grafisches Dartstellen (245) von Abhängigkeitsergebnissen;
Identifizieren eines systolischen Druckwerts (265) gemäß statistischer restlicher Dispersionswerte S2 und S3 und erhaltener regressiver Messkoeffizienten in Messfehlertoleranzen;
Ermitteln (260) von Größe von Kriterien von ermittelten Regressionskoeffizienten und restlicher Dispersionen (S2, S3), wo die Unterschiede zwischen PB-Maximalwerten weniger als der ermittelte Wert (260) der Kriterien werden und die Kontaktdruckmagnitude gleich systolischem Druck ist;
Berechnen von Pulsdruck (PP) vom systolischen und diastolischen Druck;
Durchführen von Identifizierung (285) von Werten von Amplituden früher systolischer Wellen (ASB) und später systolischer Wellen (VSB) von gemessener PB, bis zu Fragmenten (215) von diastolischem Druckmoment (RIBA);
proportionales Neuberechnen von ASB- und VSB-Werten zu den vorliegenden Karotisblutdruckeinheiten unter Verwendung ermittelter systolischer und diastolischer Blutdruckwerte;
Berechnen von Durchschnittswerten xA und xV und Standardabweichungen sA und sV von ASB- und VSB-Pulsdruckwerten gleichermaßen, aus den Durchschnittswerten Ermitteln eines maximalen Werts, dem systolischer Druckwert (265) zugeteilt ist, wo, falls der maximale Wert zu xA gehört, Wert von xA, seine Standardabweichungen sA und xV, systolischer Blutdruckwert und seine Standardabweichung sV proportional neuberechnet sind;
Suchen nach statistischem Unterschied zwischen xA- und xV-Werten (PsP) unter Verwendung von Studienzuverlässigkeitskriterien mit Wahrscheinlichkeit von weniger als 0,05, wo der Unterschied durch PP geteilt und mit 100 multipliziert ist, wodurch Augmentationsindex Alx (290) erhalten wird;
Darstellen aller Berechnungsergebnisse in einer Tabelle (295);
Aufzeichnen eines Abstandsunterschieds in Bezug auf den Vorwärtsversatz des Sensors während der Berechnung und bei dem diastolischen und systolischen Blutdruckwerten identifiziert sind.

## Revendications

1. Système de mesure de la pression sanguine au niveau de l'artère carotide et de l'onde de pouls comprenant un capteur de pression artérielle (2), un moteur commandé par programme informatique (8), et un moyen de fixation (1) destiné à fixer le dispositif de mesure (15) au cou d'un utilisateur, un moyen de traitement des données et un convertisseur A/N (60) dans lequel le moyen de fixation (1) étant un anneau rigide formé avec une fente permettant de monter le dispositif (15) sur le cou d'un utilisateur; un actionneur (10) comprenant
- un moteur commandé par programme informatique (8) commandé par un programme informatique (100) étant stocké dans un support de données lisibles par ordinateur (77) et exécuté par le moyen de traitement des données (70) étant fixé de manière rigide par des éléments de maintien (9) au moyen de fixation (1) permettant de fixer le dispositif de mesure au cou d'un utilisateur,
- un élément rotatif de forme allongée (6), muni d'un fil sur au moins une partie de sa surface externe, fixé à l'une de ses extrémités à la tige du moteur (8) par un
élément de retenue (7) permettant de fixer l'élément de forme allongée (6) à la tige du moteur (8) de sorte que l'élément de forme allongée (6) est fixé de manière rigide et axiale à la tige du moteur (8), et fixé à son autre extrémité à un moyen mobile de manière réversible (3) permettant d'effectuer un mouvement alternatif sur une voie entre le moteur (8) et l'artère, dans lequel l'élément de forme allongée (6) est configuré pour pivoter dans un élément d'écartement (5) pour délimiter son déplacement latéral et dans lequel un élément de guidage (4) étant fixé de manière rigide au moyen de fixation (1) se conformant au moins partiellement à la forme de l'au moins un moyen mobile de manière réversible (3) ; et
dans lequel le capteur (2) est fixé de manière rigide à l'extrémité libre du moyen mobile de manière réversible (3) permettant d'effectuer un mouvement alternatif sur une voie entre le moteur (8) et l'artère et étant muni d'un élément de forme convexe (42) empêchant l'artère de s'échapper du point de compression ; et
dans lequel ledit convertisseur de signal analogique-numérique (60) présente une fréquence d'échantillonnage d'au moins 1000 Hz pour l'enregistrement et le transfert de la force de pulsation mécanique.

2. Système selon la revendication 1 **caractérisé en ce que**, le moteur commandable par programme informatique (8) est un moteur pas-à-pas.

3. Système selon la revendication 1 **caractérisé en ce que**, le moteur commandable par programme informatique (8) est un servomoteur.

4. Procédé de mesure d'une pression sanguine au niveau de l'artère carotide et son onde de pouls et de calcul d'un indice d'augmentation et du diamètre interne de l'artère carotide utilisant le système selon l'une quelconque des revendications précédentes comprenant les étapes consistant à :
paramétrer une valeur de pression de contact du dispositif d'onde de pouls (15) afin de paramétrer une pression de contact qui est inférieure à la pression artérielle diastolique actuelle dans un bras ;
amener un élément de détection (41) du capteur (2) sur une artère carotide en tournant l'élément de forme allongée (6) de l'actionneur (10) du dispositif (15) de sorte que l'élément de détection (41) du capteur (2) est positionné à une distance minimale de l'artère ;
trouver des points de départ de chaque onde de pouls (PB) de l'artère carotide et enregistrer les valeurs de pression de contact de l'élément de détection (41) auxdits points de départ en utilisant le moteur commandable par programme informatique (8) ;
utiliser un programme de commande (100) permettant de choisir le temps de contrôle (110) des processus d'échantillonnages (ECG et PB), la vitesse de révolution du moteur, la valeur de l'amplitude de PB au début de PB et à son maximum et la différence entre les deux, qui est considérée comme l'amplitude de PB ;
atteindre une valeur plus petite que le niveau de pression diastolique au contact avec le capteur (2) qui est le niveau de pression principal atteint à partir du niveau de pression initial et continuer à augmenter le niveau de pression jusqu'à atteindre la valeur se situant dans la plage allant de 3 à 5 mmHg ;
arrêter le moteur (8) et enregistrer au moins trois ondes de pouls de l'artère carotide si ladite valeur située dans l'intervalle allant de 3 à 5 mmHg est atteinte ;
vérifier (120) si l'amplitude maximale de l'onde de pouls a atteint 1 à 2 mmHg du niveau de pression de contact et envoyer un signal au moteur (8) par le programme de commande (100) à des fins de rotation avant (155) si le niveau de pression de contact n'est pas atteint (130) et de mesure avant du décalage du capteur (2) et du niveau de pression de contact dans la plage allant de 3 à 5 mmHgjusqu'à la fin de la vérification ;
répéter l'opération jusqu'à ce que la valeur de l'amplitude de PB devienne inférieure à une valeur allant de 1 à 2 mmHG (160) ;
envoyer un signal du programme de commande (100) au moteur (8) à des fins de rotation arrière (170) si ladite valeur (160) est atteinte ;
échantillonner des signaux du capteur (2) dans le convertisseur de signal analogique-numérique (60), si un casque (55) R ou un électrocardiogramme est utilisé en tant que déclencheur pour initier le processus d'échantillonnage de l'ECG et l'onde de pouls (PB) ;
utiliser un support de données lisibles par ordinateur (77) pour stocker les données des valeurs initiales moyennes des ondes de pouls selon les valeurs de pression trouvées au moyen d'un programme informatique particulier (200) exécuté sur un moyen de traitement (70) qui est utilisé pour calculer au moins la quantité de décalage du capteur (2), la pression de compression, l'amplitude exacte des ondes de pouls et leurs formes, qui sont utilisées pour calculer les pressions artérielles diastolique et systolique, l'indice d'augmentation et le diamètre interne de l'artère carotide et présenter les résultats sous la forme d'une notification (300) ;
calculer l'indice d'augmentation et le diamètre interne d'une artère carotide au moyen d'un programme de calcul (200), étant exécuté dans un moyen de traitement, en réalisant les étapes consistant à :
utiliser le programme de calcul (200) pour lire (205) l'intégralité des séries d'examens du support de données (77) et fournir les données sous forme d'un graphique (210) ;
obtenir une représentation graphique (218) de l'ECG initial sélectionné et des fragments de PB (215), en l'utilisant pour sélectionner ses paramètres de temps de l'ECG et la structure d'onde de pouls, les débuts de PB (220) et les pics maximaux de PB, si lesdits paramètres de temps sont utilisés pour calculer les amplitudes de PB, qui sont des différences (230) entre tous les débuts et les valeurs maximales PB des ondes de pouls ;
réaliser un calcul adaptatif de dépendance d'une régression linéaire (240), entre les valeurs du capteur (235) des supports de données (77) et les tailles des amplitudes trouvées des ondes de pouls aux stades précoces d'examen, comprenant le calcul des valeurs de mesure, en les augmentant jusqu'à la limite (RIBA), jusqu'à ce que la pente de la courbe de dépendance régressive devienne négative, réaliser ledit calcul pour trouver la dispersion de restes ta (S1) du calcul, définissant ainsi la précision de calcul de la pression diastolique ;
calculer (250) les coefficients de dépendance de la régression linéaire et la variance résiduelle (S2) entre les valeurs de pression de contact restantes et les valeurs minimales des ondes de pouls de l'amplitude de pression de contact (235) du capteur (2) ;
réaliser des calculs (253) entre les valeurs des valeurs PB maximales restantes (225) et les valeurs de pression de contact du capteur (2), obtenant ainsi les coefficients de dépendance de la régression linéaire et une dispersion résiduelle (S3) ;
représenter graphiquement (245) les résultats de dépendance ;
identifier la valeur de pression systolique (265) selon les valeurs de dispersion résiduelles statistiques S2 et S3 et les coefficients de mesures régressives obtenus dans les marges d'erreurs de mesure ;
trouver (260) la taille des critères des coefficients de régressions trouvés et des dispersions résiduelles (S2, S3), lorsque les différences entre les valeurs PB maximales deviennent inférieures à ladite valeur trouvée (260) des critères et l'amplitude de pression de contact est égale à la pression systolique ;
calculer la pression pulsée (PP) à partir de la pression systolique et la pression diastolique ;
identifier (285) les valeurs des amplitudes des ondes systoliques précoces (ASB) et des ondes systoliques tardives (VSB) des PB mesurées, jusqu'aux fragments (215) du moment de pression diastolique (RIBA) ;
recalculer proportionnellement les valeurs ASB et VSB par rapport aux unités présentes de pression sanguine au niveau de l'artère carotide utilisant les valeurs de pressions artérielles systolique et diastolique trouvées ;
calculer en conséquence les moyennes xA et xV et les écarts-types sA et sV à partir des valeurs de pression pulsée ASB et VSB, desdites moyennes trouvant la valeur maximale, à laquelle la valeur de pression systolique (265) est attribuée, où si la valeur maximale appartient à xA, la valeur de xA, ses écarts-types sA et xV, la valeur de pression artérielle systolique et son écart-type sV sont recalculées proportionnellement ;
rechercher une différence statistique entre les valeurs xA et xV (PsP) à l'aide de critères de fiabilité de Student dont la probabilité est inférieure à 0,05, où ladite différence est divisée par PP et multipliée par 100, permettant ainsi d'obtenir l'indice d'augmentation Alx (290) ;
représenter l'ensemble des résultats de calculs dans un tableau (295) ;
enregistrer une différence de distance par rapport au décalage avant du capteur pendant le calcul et au niveau des valeurs de pressions artérielles diastolique et systolique qui sont identifiées.
